# FASCICULE DE BREVET EUROPEEN

(11) **EP 0 325 512 B1**
(45) Date de publication et mention de la délivrance du brevet: **20.01.1993**
(21) Numéro de dépôt: 89400094.2
(22) Date de dépôt: 12.01.1989
(51) Int. Cl.: C07D 233/92, C07D 405/04, A61K 31/415

(54) **Procédé de préparation d'alkyl-1 nitro-5 imidazoles**
Verfahren zur Herstellung von 1-Alkyl-5-nitro-imidazolen
Process for the preparation of 1-alkyl-5-nitro-imidazoles

(30) Priorité: 15.01.1988 FR 8800414; 10.06.1988 FR 8807773
(43) Date de publication de la demande: 26.07.1989
(73) Titulaire: RHONE-POULENC SANTE, 92160 Antony (FR)
(72) Inventeur: Bonnamas, Claude, F-03600 Commentry (FR); Massonneau, Viviane, F-69130 Ecully (FR); Mulhauser, Michel, F-69130 Ecully (FR); Rouy, Noel, F-91330 Yerres (FR)
(74) Mandataire: Pilard, Jacques

(56) Documents cités:
- EP-A- 0 043 922
- EP-A- 0 072 919
- EP-A- 0 126 183
- FR-A- 1 216 238
- FR-A- 2 263 241
- FR-A- 2 265 743
- FR-A- 2 436 780

## Description

La présente invention concerne un procédé de préparation d'alkyl-1 nitro-5 imidazoles de formule générale : 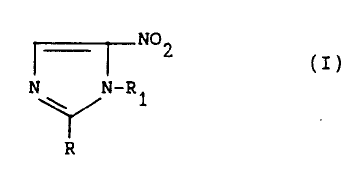
dans laquelle R représente un atome d'hydrogène ou un radical alkyle contenant 1 à 4 atomes de carbone ou alkényle contenant 2 à 4 atomes de carbone, les radicaux alkyle et alkényle étant éventuellement substitués par un ou plusieurs radicaux, identiques ou différents, choisis parmi les radicaux phényles, phénoxy ou hétérocycliques oxygénés à 5 ou 6 chaînons, ou bien un radical aryle contenant 6 à 10 atomes de carbone éventuellement substitué par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, ou bien un radical cycloalkyle contenant 5 ou 6 atomes de carbone, les radicaux phényles, phénoxy ou hétérocycliques pouvant être éventuellement substitués par un ou plusieurs atomes ou radicaux, identiques ou différents, choisis parmi les atomes d'halogène et les radicaux alkyles contenant 1 à 4 atomes de carbone, alkoxy dont la partie alkyle contient 1 à 4 atomes de carbone, phényle, phénoxy ou nitro, et R₁ représente un radical alkyle contenant 1 à 12 atomes de carbone en chaîne droite ou ramifiée.

Dans le brevet français FR 75 09065 (2 265 743) a été décrit un procédé de préparation des dérivés de l'imidazole de formule générale (I) par action d'un sulfate d'alkyle de formule générale :

R₁O-SO₂-OR₁ (II)

dans laquelle R₁ est défini comme précédemment sur un dérivé de l'imidazole de formule générale : 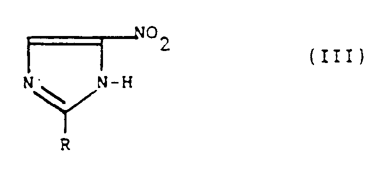
dans laquelle R est défini comme précédemment, en présence d'un acide carboxylique.

Il a maintenant été trouvé, et c'est ce qui fait l'objet de la présente invention, que les dérivés de l'imidazole de formule générale (I) peuvent être obtenus par action d'un sulfate d'alkyle de formule générale (II) sur un dérivé de l'imidazole de formule générale : 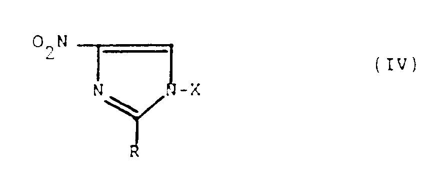
dans laquelle R est défini comme précédemment et X représente un groupement facilement éliminable par hydrolyse, en opérant dans un solvant organique, suivie de l'hydrolyse ou de l'alcoolyse éventuellement in situ.

Dans la formule générale (IV), X représente un radical hydroxyméthyle, alkoxyméthyle dont la partie alcoyle contient 1 à 4 atomes de carbone, acyloxyméthyle dont la partie acyle contient 1 à 4 atomes de carbone, un radical éthylénique allylique, tel que le radical allyle, ou un radical arylméthyle tel que le radical benzyle.

Généralement le procédé est mis en oeuvre à une température comprise entre 60 et 120°C, de préférence voisine de 80°C.

Comme solvant organique, on utilise de préférence des esters (acétate de méthyle, acétate d'éthyle), des éthers (méthyltertiobutylether) ou des hydrocarbures aliphatiques ou aromatiques eventuellement halogénés (xylène, chlorure de méthylène).

Généralement, l'hydrolyse ou l'alcoolyse du produit de condensation est effectuée par chauffage dans l'eau ou un alcool (méthanol, éthanol) à une température comprise entre 60 et 100°C. Il n'est pas nécessaire d'isoler le produit de condensation préalablement à l'hydrolyse.

Les dérivés de l'imidazole de formule générale (IV) peuvent être préparés dans les conditions décrites dans le brevet anglais GB 1 026 631.

Les dérivés de l'imidazole de formule générale (I) présentent des propriétés thérapeutiques ou constituent des intermédiaires pour la préparation de colorants, d'adjuvants pour textiles ou d'insecticides.

Les exemples suivants, donnés à titre non limitatif, montrent comment l'invention peut être mise en pratique.

### EXEMPLE 1

Dans un ballon muni d'un agitateur et d'une ampoule de coulée, on introduit 3,98 g (0,02 mole) d'acétoxyméthyl-1 méthyl-2 nitro-4 imidazole et 10 cm3 de xylène. Le mélange est chauffé à 80°C puis on introduit 3,2 g (0,024 mole) de sulfate de méthyle. On chauffe à 80°C pendant 2 heures. On ajoute alors 25 cm3 d'eau puis chauffe à 80°C pendant 2 heures 30 minutes.

Après dilution, l'analyse du milieu réactionnel par chromatographie liquide à haute performance montre qu'il contient 2,8 g de diméthyl-1,2 nitro-5 imidazole.

Le taux de transformation du méthyl-2 nitro-4 (ou -5) imidazole est voisin de 100%.

Le rendement en diméthyl-1,2 nitro-5 imidazole est supérieur à 95% par rapport au méthyl-2 nitro-4 (ou -5) imidazole mis en oeuvre.

### EXEMPLE 2

Dans un ballon tricol de 50 cm3, on introduit 20 cm3 de xylène puis on ajoute 3,7 g d'acétoxyméthyl-1 nitro-4 imidazole puis on chauffe à 80°C. On observe la "fusion" de l'acétoxyméthyl-1 nitro-4 imidazole et la formation de deux phases. On ajoute, en 30 minutes à 80°C, 2,52 g de sulfate de diméthyle. Le mélange réactionnel, constitué de 2 phases incolores, est chauffé à 80°C pendant 1 heure. Après refroidissement à 70°C, on ajoute 20 cm3 d'eau et agite pendant 3 heures 30 minutes à cette température.

Le dosage du mélange réactionnel par chromatographie liquide à haute performance (CLHP) montre que :
- le taux de transformation de l'acétoxyméthyl-1 nitro-4 imidazole est de 92 %
- le rendement en méthyl-1 nitro-5 imidazole est de 83 %.

### EXEMPLE 3

On opère comme dans l'exemple 2 mais à une température de 100°C. Après hydrolyse pendant 1 heure 30 minutes au reflux puis refroidissement à une température voisine de 20°C, la phase aqueuse est séparée par décantation et la phase xylénique est lavée par 3 fois 20 cm3 d'eau.

Les phases aqueuses réunies, dosées par CLHP montrent que :
- le taux de transformation de l'acétoxyméthyl-1 nitro-4 imidazole est de 94,6 %
- le rendement en méthyl-1 nitro-5 imidazole est de 87,2 %.

## Revendications

1. Procédé de préparation d'alkyl-1 nitro-5 imidazoles de formule générale : 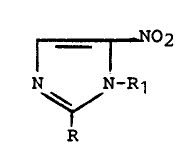 dans laquelle R représente un atome d'hydrogène ou un radical alkyle contenant 1 à 4 atomes de carbone et R₁ représente un radical alkyle contenant 1 à 12 atomes de carbone, caractérisé en ce que l'on fait agir un sulfate d'alkyle de formule générale :
R₁O-SO₂-OR₁
dans laquelle R₁ est défini comme précédemment sur un dérivé de l'imidazole de formule générale : 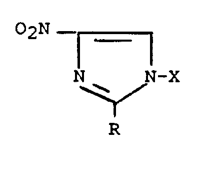 dans laquelle R est défini comme précédemment et X représente un radical hydroxyméthyle, alkoxyméthyle contenant 1 à 4 atomes de carbone, acyloxyméthyle contenant 1 à 4 atomes de carbone, allyle ou benzyle en opérant dans un solvant organique, à une température comprise entre 60 et 120°C, puis hydrolyse à chaud, éventuellement in situ, le produit obtenu.

2. Procédé selon la revendication 1 caractérisé en ce que le solvant est choisi parmi les esters, les éthers et les hydrocarbures aliphatiques ou aromatiques éventuellement halogénés.

## Patentansprüche

1. Verfahren zur Herstellung von 1-Alkyl-5-nitroimidazolen der allgemeinen Formel 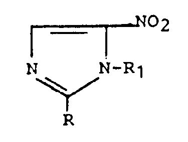 worin R für ein Wasserstoffatom oder einen Alkylrest mit 1 bis 4 Kohlenstoffatomen steht und R₁ einen Alkylrest mit 1 bis 12 Kohlenstoffatomen bedeutet, dadurch gekennzeichnet, daß man ein Alkylsulfat der allgemeinen Formel
R₁O-SO₂-OR₁
worin R₁ wie vorstehend definiert ist, mit einem Imidazolderivat der allgemeinen Formel 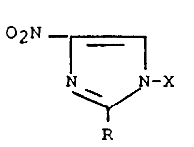 worin R wie vorstehend definiert ist und X einen Hydroxymethyl-, Alkoxymethyl- mit 1 bis 4 Kohlenstoffatomen, Acyloxymethyl- mit 1 bis 4 Kohlenstoffatomen, Allyl- oder Benzylrest bedeutet, umsetzt, wobei man in einem organischen Lösungsmittel bei einer Temperatur zwischen 60 und 120°C arbeitet, wonach man in der Wärme,gegebenenfalls in situ, das erhaltene Produkt hydrolysiert.

2. Verfahren gemäß Anspruch 1, dadurch gekennzeichnet, daß das Lösungsmittel unter den Estern, den Ethern und den gegebenenfalls halogenierten, aliphatischen oder aromatischen Kohlenwasserstoffen ausgewählt wird.

## Claims

1. Process for preparing 1-alkyl-5-nitroimidazoles of general formula: 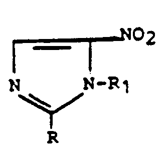 in which R represents a hydrogen atom or an alkyl radical containing 1 to 4 carbon atoms and R₁ represents an alkyl radical containing 1 to 12 carbon atoms, characterised in that an alkyl sulphate of general formula:
R₁O-SO₂-OR₁
in which R₁ is defined as above, is reacted with an imidazole derivative of general formula: 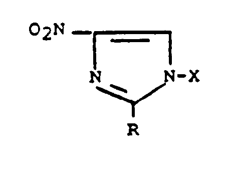 in which R is defined as above and X represents a hydroxymethyl, alkoxymethyl radical containing 1 to 4 carbon atoms, an acyloxymethyl radical containing 1 to 4 carbon atoms, or an allyl or benzyl radical, in an organic solvent at a temperature of between 60 and 120°C and in that the product obtained is then hydrolysed while warm, optionally in situ.

2. Process according to Claim 1, characterised in that the solvent is chosen from optionally halogenated, aromatic or aliphatic hydrocarbons, ethers and esters.
